# EUROPEAN PATENT APPLICATION

(11) **EP 2 826 502 A1**
(43) Date of publication of application: **21.01.2015**
(21) Application number: 13761674.4
(22) Date of filing: 12.02.2013
(51) Int. Cl.: A61M 1/02, A61M 5/14

(54) **BLOOD-FLOW-PATH CONNECTING DEVICE AND BLOOD-BAG SYSTEM**

(30) Priority: 12.03.2012 JP 2012054338
(71) Applicant: Terumo Kabushiki Kaisha, Tokyo 151-0072 (JP)
(72) Inventor: KANAMOTO, Kazuaki, Ashigarakami-gun Kanagawa 259-0151 (JP)
(74) Representative: TBK
(86) International application number: PCT/JP2013/053191
(87) International publication number: WO 2013/136889

(57) **Abstract**

In a blood-flow-path connector (10) and blood bag system (12), a blocking part (74) is provided in a hollow section (68c) in a tubular body (68), and a communicating part (70) that is axially movable within the hollow section (68c) is disposed. By moving the communicating part (70) relative to the tubular body (68) in a pushing direction, the blocking part (74) is penetrated or pressed to be opened; thus, a flow-path in the tubular body (68) and a flow-path in the communicating part (70) come to communicate with each other.

## Description

### Technical Field

The present invention relates to a blood-flow-path connector to be used to secure a blood-flow-path and a blood bag system having the same.

### Background Art

Conventionally, blood collection from a blood donor for blood donation and the like using blood collection equipment has been practiced by puncturing the blood donor by inserting a blood collection needle through which blood of the blood donor is drawn and then introduced into a blood collection bag. In this, before puncturing the blood donor with the blood collection needle, the puncture site in the blood donor is cleansed with alcohol or the like to prevent bacteria from entering the collected blood; nevertheless, there still have been cases where the collected blood is contaminated with bacteria present on and beneath skin. Upon accidental bacterial invasion in the collected blood, some types of bacteria even proliferate in the blood during storage in a blood collection bag or specimen bag. If such blood contaminated with bacteria is used for transfusion in other patients, infections can be caused, potentially leading to severe conditions.

Thus, for the purpose of protecting collected blood from bacterial contamination, specifically of eliminating the first volume of drawn blood during blood collection, blood collection equipment has been provided, wherein a first flow-path for introducing collected blood into a blood collection bag and a second flow-path for eliminating the initially-drawn blood are connected via a branch part, and a sealing member is provided between the branch part and the blood collection bag, which sealing member blocks the flow-path in an initial state while it opens the flow-path upon opening operation (see, for example, Japanese Patent Publication No. 3776227).

This sealing member is partially breakable, and while it blocks the first flow-path to keep the initially-drawn blood from entering the blood collection bag before breakage, it can be partially broken to open the first flow-path so that the collected blood can flow into the blood collection bag.

### Summary of Invention

### Technical Problem

However, in the blood collection equipment according to Japanese Patent Publication No. 3776227, upon opening the first flow-path, a breakable part in the sealing member is broken to separate a solid column-like part, requiring a great amount of force for the breakage operation; hence, the fore-and-aft flow-path cannot be opened with ease. In other words, a user cannot switch between the flow-path in the blood collection equipment with easy operation. Further, sometimes the breakage of the breakable part is incomplete, resulting in a failure to secure a flow-path large enough to pass blood smoothly, which potentially impairs blood flow leading to hemolysis.

### Solution to Problem

The present invention has been made taking the above problems into consideration with an objective of providing a blood-flow-path connector for connecting fore-and-aft flow-path by simple operation and securing an appropriate blood-flow-path at a connecting part, and a blood bag system having the blood-flow-path connector.

For the purpose of achieving the above mentioned objective, a blood-flow-path connector according to the present invention is characterized by having a tubular body and a communicating part that have a hollow configuration with a flow-path in the hollow section for a fluid passing in an axial direction therein, wherein: the tubular body has a first end constituting one end and a second end constituting the other end; a direction from the second-end side of the tubular body to the first-end side is defined as a pushing direction; the hollow section in the tubular body is provided with a blocking part for blocking the fluid flowing in the flow-path in the hollow section; the communicating part is disposed on the second-end side of the tubular body, axially movable in the hollow section of the tubular body; the blocking part opens as the communicating part moves relative to the tubular body in the pushing direction, and due to the opening of the blocking part, the flow-path in the tubular body and the flow-path in the communicating part come to communicate with each other.

According to the above configuration, the communicating part is axially movable in the hollow section in the tubular body where the blocking part is provided; therefore, by simply pushing the communicating part relative to the tubular body, the flow-path before and after the blood-flow-path connector can be easily connected; moreover, because the movement of the communicating part relative to the tubular body opens the flow-path, an adequately large flow-path at the connecting part can be secured.

In the above blood-flow-path connector, preferably, a hollow section in the communicating part is the flow-path; one end of the communicating part that constitutes a blocking-part side is formed in such a configuration that it can penetrate the blocking part; the blocking part is penetrated and thereby opened by the communicating part that has moved relative to the tubular body in a pushing direction. With this configuration in which the communicating part penetrates the blocking part, operation for connecting the flow-path becomes easier, and a more adequate flow-path can be secured.

In the above blood-flow-path connector, preferably, a hollow cover part that is axially contractible and capable of holding the communicating part is provided; a first end of the cover part is connected to the tubular body liquid-tightly; a second end of the cover part is connected to the communicating part liquid-tightly and is movable together with the communicating part in an axial direction. With this cover part, the communicating part can be kept clean, and even when a liquid leaks from the communicating part, the liquid is kept from leaking externally.

In the above blood-flow-path connector, preferably, the cover part is formed into a bellows. By forming the cover part into a bellows, the cover part is axially contractible with more ease, greatly reducing operation force required for flow-path connecting operation.

In the above blood-flow-path connector, preferably, the communicating part is formed into a shape enabled to press the blocking part; the tubular body includes a fitting part for tightly fitting to the blocking part at a predetermined position in an axial direction; the blocking part has a slit that axially penetrates the blocking part, and is adapted to be axially movable in the tubular body, and when moved in a pushing direction to be disjoined from the fitting part by means of the communicating part which has moved relative to the tubular body in the pushing direction, the slit is opened to open the blocking part. Thus, with the configuration in which the slit in the blocking part is opened when the communicating part presses the blocking part, flow-path can be connected with easier operation; this also eliminates a need for breakage for opening, whereby a flow-path can be secured more reliably.

In the above blood-flow-path connector, preferably, the tubular body, in its hollow section, includes a hollow sliding part that is axially slidable within the hollow section and a hollow contracting part that is axially contractible, wherein an end of the sliding part on the second-end side of the tubular body is the blocking part, and the sliding part and the contracting part's respective hollow sections consist a flow-path in the tubular body. With the contracting part and sliding part, regardless of the sliding of the blocking part, a flow-path in the tubular body can be secured. Further, the sliding part is capable of supporting the sliding and/or deformation of the blockingpart, while the contractingpart is capable of restoring the sliding part and the blocking part to their initial states.

In the above blood-flow-path connector, preferably, a hollow operating part that can hold the communicating part is provided; the operating part is movably and axially screwed onto the tubular body; the communicating part is pivotally supported by the operating part and moves in response to the movement of the operating part relative to the tubular body. By simply rotating this cover part around the tubular body, the cover part is tightened against the tubular body in a pushing direction, and the communicating part pushes the blocking part, opening the slit to open the blocking part; thus, the flow-path can be connected with easier operation. Further, because the positions of the cover part and the communicating part relative to the tubular body can be kept, a connected state can be stably sustained.

Furthermore, a blood bag system according to the present invention includes bags for containing blood or blood components and tubes to serve as flow-path to the bags for the blood or blood components, wherein a blood-flow-path connector is disposed in the tube(s), and the blood-flow-path connector is characterized by having a tubular body and a communicating part that have a hollow configuration with a flow-path in the hollow section for a fluid passing in an axial direction therein, wherein: the tubular body has a first end constituting one end and a second end constituting the other end; a direction from the second-end side of the tubular body to the first-end side is defined as a pushing direction; the hollow section in the tubular body includes a blocking part for blocking the fluid flowing in the flow-path in the hollow section; the communicating part is disposed on the second-end side of the tubular body, axially movable in the hollow section of the tubular body; the blocking part opens as the communicating part moves relative to the tubular body in the pushing direction, and due to the opening of the blocking part, the flow-path in the tubular body and the flow-path in the communicating part come to communicate with each other.

According to the above configuration, by simply pushing the communicating part relative to the tubular body, the flow-path before and after the blood-flow-path connector can be connected with ease, allowing the blood flow in the blood-flow-path tubes disposed in the blood bag system to be switched with ease. Further, because a flow-path opens due to the movement of the communicating part relative to the tubular body, an adequately large flow-path can be secured at the connecting part.

In the above blood bag system, preferably, the bags include a blood collection bag for containing blood drawn from a donor and a initially-drawn blood bag for containing initially-drawn blood during blood collection; among the tubes, a first Blood collection tube to be connected to a blood collection needle to be punctured into the donor is branched into a second Blood collection tube to be connected to the blood collection bag and a branch tube to be connected to the initially-drawn blood bag, wherein the blood-flow-path connector is disposed in the second Blood collection tube. By disposing the blood-flow-path connector in the second Blood collection tube, the initially-drawn blood is reliably kept from flowing into the blood collection bag, while connection of blood-flow-paths for containing blood in the blood collection bag can be performed with ease.

### Advantageous Effects of Invention

According to the present invention, in the blood bag system, the fore-and-aft flow-path can be connected with easy operation, and an adequate blood-flow-path at the connecting part therein can be secured.

### Brief Description of Drawings

Fig. 1 is an overall structural view showing a blood bag system having a blood-flow-path connector according to a first embodiment of the present invention.
Fig. 2 is a perspective view of the blood-flow-path connector shown in Fig. 1.
Fig. 3 is a sectional side view showing a state before opening of a blocking part in the blood-flow-path connector shown in Fig. 1.
Fig. 4 is a sectional side view showing a state after opening of the blocking part in the blood-flow-path connector shown in Fig. 1.
Fig. 5 is a sectional side view showing a state before opening of the blocking part in a modification of a cover part in the blood-flow-path connector shown in Fig. 1
Fig. 6 is a sectional side view showing a state after opening of the blocking part in a modification of the cover part in the blood-flow-path connector shown in Fig. 1
Fig. 7 is a sectional side view showing a state before opening of a blocking part in a blood-flow-path connector according to a second embodiment of the present invention.
Fig. 8 is a sectional side view showing a state after opening of a blocking part in the blood-flow-path connector according to the second embodiment of the present invention.

### Description of Embodiments

A blood-flow-path connector according to the present invention will now be described in detail by providing preferable embodiments and referring to accompanied drawings. It should be noted that, for the convenience of explanation, dimensional proportion between components in individual drawings and dimensional proportion between the same components across several drawings have been appropriately modified but do not necessarily coincide with actual proportion.

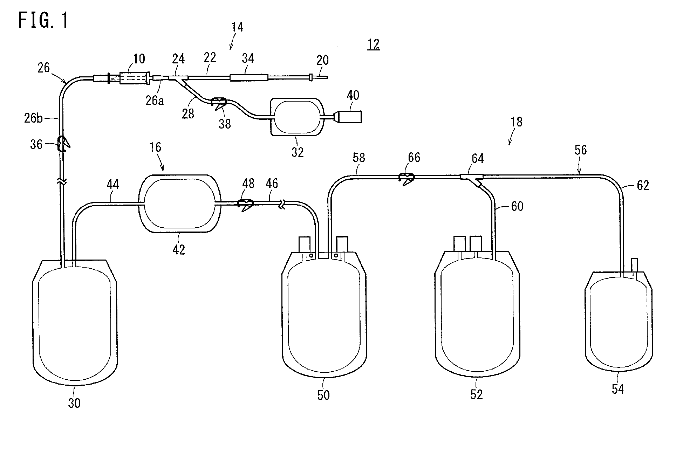

Fig. 1 is an overall structural view of a blood bag system 12 having a blood-flow-path connector 10 according to a first embodiment of the present invention. This blood bag system 12 is for separating blood containing multiple components into different multiple components and distributing each of the components into different bags to contain and store the components.

The blood bag system 12 has a the blood collection equipment 14 for collecting blood (whole blood) from a blood donor (donor) ; a pre-processing part 16 for eliminating a predetermined blood component from the whole blood; and a separation processing part 18 for separating residual blood components after the predetermined component has been removed into multiple blood components and for containing each of the components in different bags, wherein the blood collection equipment 14 includes the blood-flow-path connector 10.

The blood collection equipment 14 has a blood collection needle 20; a first Blood collection tube 22; a branch part 24; a second Blood collection tube 26; a branch tube 28; a blood collection bag 30; and a initially-drawn blood bag 32, wherein the blood-flow-path connector 10 is disposed in a middle section of the second Blood collection tube 26. For example, the second Blood collection tube 26 consists of a first tube 26a on the branch part 24 side and a second tube 26b on the blood collection bag 30 side, and the blood-flow-path connector 10 is disposed between the first tube 26a and the second tube 26b.

The blood collection needle 20 is punctured into a donor for collecting blood, is connected to one end of the first Blood collection tube 22, and after use, it is protected by a needle guard 34 that is movable along a longitudinal direction of the first Blood collection tube 22.

The first Blood collection tube 22 connects to one end of the second Blood collection tube 26 and one end of the branch tube 28 via the branch part 24, and the other end of the second Blood collection tube 26 and the other end of the branch tube 28 connect to the blood collection bag 30 and the initially-drawn blood bag 32, respectively. In a middle section of the second Blood collection tube 26 and the branch tube 28, clamps 36 and 38 for blocking and releasing the flow-path in the second Blood collection tube 26 and the branch tube 28, respectively, are provided.

The blood collection bag 30 is a bag for containing blood (whole blood) collected from a donor. Further, the blood collection bag 30 connects to the pre-processing part 16 via an entrance-side tube 44.

The initially-drawn blood bag 32 connects to a sampling port 40; by attaching a Blood collection tube (not shown) to the sampling port 40, initially-drawn blood is collected in the Blood collection tube (not shown), which collected initially-drawn blood will be subjected to blood testing.

The blood collection bag 30 and the initially-drawn blood bag 32 are, for example, configured in a bag shape by lapping a flexible sheet material made of soft resin such as polyvinyl chloride or polyolefin one on top of each other, and then fusing or bonding the edges.

When collecting blood from a donor using this the blood collection equipment 14, prior to containing blood in the blood collection bag 30, first, a first volume of drawn blood is collected in the initially-drawn blood bag 32 by a predetermined amount. Here, by bringing a clamp 38 into an open state with the blood-flow-path connector 10 kept in a closed state (an initial state), the initially-drawn blood is kept from entering the second Blood collection tube 26 side, i.e., the blood collection bag 30 side; this also enables for the initially-drawn blood to flow into the initially-drawn blood bag 32 via the first Blood collection tube 22, the branch part 24, and the branch tube 28.

When containing collected blood in the blood collection bag 30, the blood-flow-path connector 10 is brought into an open state (a connected state), while the clamp 36 is brought into an open state and the clamp 38 is brought into a closed state, whereby the collected blood is allowed to flow into the blood collection bag 30 via the first Blood collection tube 22, the branch part 24, and the second Blood collection tube 26. After the blood has flown into the blood collection bag 30, the clamp 36 is brought into the closed state.

The pre-processing part 16 has a filter 42 for eliminating a predetermined blood component from whole blood; the entrance-side tube 44 whose one end is connected to the blood collection bag 30 and the other end is connected to an entrance of the filter 42; and an exit-side tube 46 whose one end is connected to an exit of the filter 42 and the other end is connected to the separation processing part 18.

The filter 42 is, for example, a leukocyte removing filter for eliminating leucocytes as a predetermined blood component. Also, the filter 42 may be one that eliminates platelets too.

The entrance-side tube 44 is a tube for transferring blood contained in the blood collection bag 30 to the filter 42.

The exit-side tube 46 is a tube for transferring residual blood components after the predetermined blood component has been eliminated by the filter 42 to the separation processing part 18. In a middle section of the exit-side tube 46, the clamp 48 that blocks and releases the flow-path of the exit-side tube 46 is provided.

The separation processing part 18 has a first bag 50 for containing residual blood components after the predetermined blood component has been eliminated by the filter 42; a second bag 52 for containing a supernatant such as plasma or the like that is obtained by separating the blood components in the first bag 50; a third bag 54 for containing a red-blood-cell preserving solution; and a transfer line 56 connected to the first bag 50, the second bag 52, and the third bag 54.

Similar to the blood collection bag 30, the first bag 50, the second bag 52, and the third bag 54 may be configured by forming a flexible sheet material made of soft resin into a bag shape.

The first bag 50 serves both as a bag for containing residual blood components after a predetermined blood component has been eliminated by the filter 42, and as a bag for containing precipitated components, such as concentrated red blood cells, to be obtained by separating the residual blood components.

The red-blood-cell preserving solution to be contained in the third bag 54 is, for example, MAP, SAGM, or OPTISOL.

The transfer line 56 has a first transfer tube 58 connected to the first bag 50; a second transfer tube 60 connected to the second bag 52; a third transfer tube 62 connected to the third bag 54; a branch part 64 for connecting the first, second, and third transfer tubes 58, 60, and 62; and a clamp 66 disposed in a middle section of the first transfer tube 58 for blocking and releasing the flow-path in the first transfer tube 58.

Incidentally, the tubes in the blood bag system 12 are flexible, transparent resin tubes. The clamps may be standard products for conventional use. All clamps are in an open state during sterilization and storage of the blood bag system 12 prior to use; hence, the inside of the bags are in communication with each other in a uniform antiseptic state.

The blood-flow-path connector 10 according to the first embodiment will now be explained.

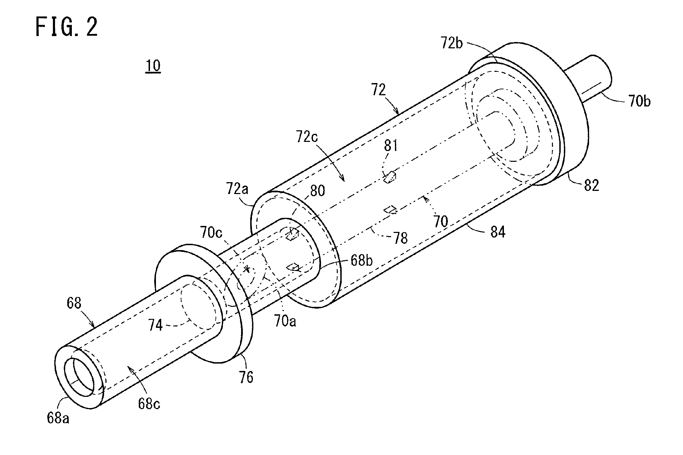

Fig. 2 is a perspective view of the blood-flow-path connector 10 according to the first embodiment. The blood-flow-path connector 10 includes a tubular body 68 having the blocking part 74; a communicating part 70 for opening the blocking part 74; and a cover part 72 for covering the communicating part 70.

The tubular body 68 is formed having a hollow cylindrical shape, whose hollow section 68c serves as a blood-flow-path, and has a first end 68a constituting one end and a second end 68b constituting the other end; the hollow section 68c includes the blocking part 74 in a middle section thereof for blocking the flow-path. The tubular body 68 is configured so that the communicating part 70 is axially movable within the hollow section 68c, and an inner circumferential surface of the hollow section 68c has an inner diameter slightly greater than an outer diameter of the communicating part 70.

The first end 68a and the second end 68b of the tubular body 68 are both open relative to an axial direction. The first end 68a of the tubular body 68 is configured so that it can connect to a tube as a blood-flow-path; for example, it is connected to the second tube 26b that is on the blood collection bag 30 side of the second Blood collection tube 26. The second end 68b of the tubular body 68 is connected to a first end 72a of the cover part 72.

A non-limiting example of constituent material for the tubular body 68 is a resin material. The tubular body 68 may be configured substantially transparent so as to ensure the inner visibility.

The tubular body 68 may be configured to have an outwardly protruded flange 76 on an outer circumference thereof to enable a user to put a finger thereon to facilitate operation. The flange 76 may be disposed at the same position as the blocking part 74 in the axial direction of the tubular body 68, or may be disposed at a different position.

Alternatively, the tubular body 68 may be configured with, for example, a ring-like seal (not shown) on an inner circumferential surface near the blocking part 74 such that when the communicating part 70 penetrates the blocking part 74, this seal seals a gap between the inner circumferential surface of the tubular body 68 and the outer circumferential surface of the communicating part 70.

The blocking part 74 is for blocking a flow-path liquid-tightly at a middle section of the hollow section 68c in the tubular body 68, and is adapted to have, for example, a membrane or partition wall that divides the flow-path before and after itself. The blocking part 74 is opened due to the movement of the communicating part 70 in a direction from the second end 68b side of the tubular body 68 to the first end 68a side (hereafter, also called "pushing direction").

The blocking part 74 in the first embodiment is provided integrally with the tubular body 68 in a middle section of the hollow section 68c in the tubular body 68, which is opened by being penetrated by the communicating part 70 that moves relative to the tubular body 68 in a pushing direction, whereby the fore-and-aft flow-path in the tubular body 68 become connected.

For a constituent material for the blocking part 74, there is no particular limitation as long as one can be penetrated by the communicating part 70, that is, a material that is softer than the communicating part 70; for example, a resilient material or soft resin material is used preferably.

Further, the blocking part 74 may be configured so that, upon being penetrated by the communicating part 70, a broken part thereof serves as a seal to seal a gap between an inner circumferential surface of the tubular body 68 and an outer circumferential surface of the communicating part 70.

The communicating part 70 has an axially open, hallow shape, whose hollow section 70c serves as a blood-flow-path, and a first end 70a and a second end 70b thereof are both open relative to an axial direction. The communicating part 70 is provided on the second end 68b side of the tubular body 68; the first end 70a of the communicating part 70 constitutes the blocking part 74 side, and is disposed on the pushing direction side relative to the second end 70b.

The communicating part 70 is configured so that when it is inserted from the second end 68b side into the hollow section 68c in the tubular body 68, it is axially movable within the hollow section 68c in the tubular body 68; hence has an outer diameter slightly smaller than an inner diameter of the hollow section 68c. The communicating part 70 acts on the blocking part 74 as it moves relative to the tubular body 68 in a pushing direction; for example, it can open the blocking part 74 by means of penetrating the blocking part 74.

The communicating part 70 in the first embodiment is capable of penetrating the blocking part 74 in the tubular body 68, and includes a main body 78 that penetrates the blocking part 74 on the first end 70a side. Here, the first end 70a of the communicating part 70 is a distal end of the main body 78, which distal end having a suitable shape for penetrating the blocking part 74; for example, the distal end may be shaped like a sharp needle distal end. The second end 70b of the communicating part 70 is configured so that it can connect to a tube as a blood-flow-path; for example, it is connected to the first tube 26a that is on the branch part 24 side in the second Blood collection tube 26.

For a constituent material for the communicating part 70, there is no particular limitation as long as one is capable of penetrating the blocking part 74, that is, a material that is harder than the blocking part 74; for example, a hard resin material is used preferably. The communicating part 70 may be configured substantially transparent so as to ensure the inner visibility.

Preferably, the communicating part 70 includes the support parts 80 and 81 on an outer circumference thereof for supporting the communicating part 70 against the tubular body 68 or the cover part 72. The support parts 80 and 81 may be, for example, a simple projetion outwardly protruding from a predetermined position on an outer circumference of the communicating part 70, or a tapered ring-shaped part, circumferentially extending on the outer circumference of the communicating part 70 and outwardly protruding in a manner that its outer diameter increases toward the second end 70b side, or the like.

Preferably, the support part 80 provided on the first end 70a side of the communicating part 70 is adapted to engage the tubular body 68 in an initial state prior to penetration of the blocking part 74, and engage the blocking part 74 in a connected state after penetration of the blocking part 74. Preferably, the support part 81 provided on the second end 70b side of the communicating part 70 is adapted to engage the tubular body 68 in a connected state after penetration of the blocking part 74.

Thus, the support part 80 temporarily prevents the communicating part 70 frommoving axially relative to the tubular body 68 and the cover part 72 in an initial state prior to penetration of the blocking part 74. Also, the support parts 80 and 81 temporarily prevent the communicating part 70 from moving axially relative to the tubular body 68 and the cover part 72, respectively, in a connected state after penetration of the blocking part 74. Hence, the support parts 80 and 81 act as safety locks for the communicating part 70 relative to the tubular body 68 and the cover part 72.

Engagement of the support part 80 to the tubular body 68 has a certain degree of strength that is, at least strong enough that the communicating part 70 does not come out of the cover part 72 and the tubular body 68. Engagement of the support part 80 to the blocking part 74 has a certain degree of strength that is, at least strong enough that the communicating part 70 is not allowed to move relative to the tubular body 68 towards an opposite direction to a pushing direction and that a user cannot easily pull out the communicating part 70 from the tubular body 68.

Further, the communicating part 70 may be configured to have an outwardly protruding base 82 on an outer circumference thereof to enable a user to put a finger thereon to facilitate operation. The base 82 is preferably disposed between the main body 78 and the second end 70b.

The cover part 72 is a bag-like article having an axially open, hallow shape, and covers the communicating part 70 in a manner that the communicating part 70 is held in its hollow section 72c; and is provided on the second end 68b side of the tubular body 68. The cover part 72, for example, may have a hollow cylindrical shape having the same axial direction as the blood-flow-path connector 10, as shown in Fig. 2, or, may have an ellipsoidal or spherical shape.

The first end 72a of the cover part 72 is adapted to have an opening where the main body 78 of the communicating part 70 is inserted therethrough; and is connected to the tubular body 68 liquid-tightly, for example, is connected to the second end 68b of the tubular body 68. A second end 72b of the cover part 72 is connected to the communicating part 70 liquid-tightly, for example, is connected to the base 82 or the like on the second end 70b side of the communicating part 70; also, the second end 72b of the cover part 72 is adapted to be axially movable together with the communicating part 70. Thus, the cover part 72 keeps the communicating part 70 clean, and even when a liquid leaks from the communicating part 70, the liquid is kept from leaking externally.

An outer circumferential part of the cover part 72, that is a main body 84 excluding the first end 72a and the second end 72b of the cover part 72, is configured to be axially contractible.

For a constituent material for the cover part 72, there is no particular limitation as long as one is flexible enough to be able to contract axially; for example, a resilient material or soft resin material is used preferably. The cover part 72 may be configured substantially transparent so as to ensure the inner visibility.

The blood-flow-path connector 10 according to an embodiment of the present invention is basically configured as described above; hereafter, the action and effect thereof will be explained referring to examples where the blood-flow-path connector 10 is used for connecting the fore-and-aft blood-flow-paths.

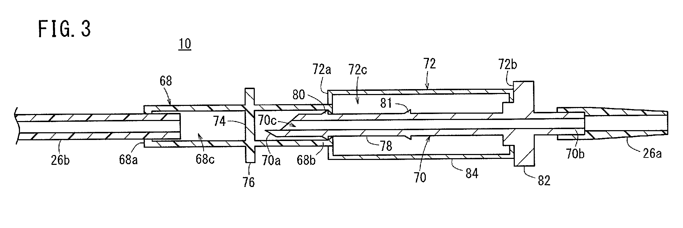

First, as shown in Fig. 3, in an initial state of the blood-flow-path connector 10 before the communicating part 70 penetrates the blocking part 74 of the tubular body 68, flow-path before and after the blocking part 74 are divided by the blocking part 74, whereby the blood flow from the Blood collection tube 22 to the second Blood collection tube 26 has been blocked.

Here, the communicating part 70 has its support part 80 in engagement with the tubular body 68 to prevent escaping from the cover part 72 and the tubular body 68.

Next, a user pushes the communicating part 70 relative to the tubular body 68 in a pushing direction in order to make blood flow from the first Blood collection tube 22 to the second Blood collection tube 26.

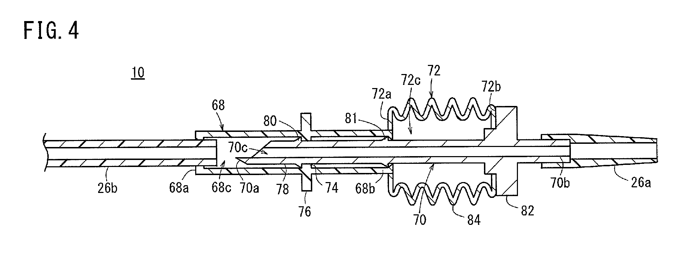

Here, as shown in Fig. 4, the main body 84 of the cover part 72 contracts axially, reducing a distance between the first end 72a and the second end 72b thereof.

The main body 78 of the communicating part 70 moves within the hollow section 68c in the tubular body 68 in a pushing direction, eventually breaking and penetrating the blocking part 74 of the tubular body 68. As a result, the blocking part 74 is opened and the flow-path before and after the blocking part 74 are connected; specifically, the hollow section 70c in the communicating part 70 and the hollow section 68c in the tubular body 68 come to communicate with each other.

In the communicating part 70, at least one of the following engagements is made: engagement of the support part 80 on the first end 70a side with the blocking part 74 and engagement of the support part 81 on the second end 70b side with the tubular body 68, such that the communicating part 70 is supported by the tubular body 68 while a communicated state between the hollow section 70c in the communicating part 70 and the hollow section 68c in the tubular body 68 is sustained.

Thus, the first Blood collection tube 22 and the second Blood collection tube 26 come to communicate with each other, having blood flow from the first Blood collection tube 22 to the second Blood collection tube 26.

As described above, in the blood-flow-path connector 10 of this embodiment, by simply pushing the communicating part 70 relative to the tubular body 68 in a pushing direction, flow-path before and after the blood-flow-path connector 10 can be connected; since this connection is made by communicating the hollow section 70c in the communicating part 70 and the hollow section 68c in the tubular body 68, smooth flow of blood is facilitated; thus, the fore-and-aft flow-path can be connected with easy operation, and an adequate blood-flow-path at the connection can be secured.

The above-mentioned embodiment illustrates a case where the blood-flow-path connector 10 is disposed in a middle section of the second Blood collection tube 26; alternatively, the blood-flow-path connector 10 may be disposed between the branch part 24 and the second Blood collection tube 26 for connecting the second end 70b of the communicating part 70 to the branch part 24, for example.

The blood-flow-path connector 10 of the present invention may be disposed in the other tubes in the blood bag system 12. For example, the blood-flow-path connector 10 may be disposed on an end on the blood collection bag 30 side of the entrance-side tube 44 in the pre-processing part 16 to temporarily keep blood in the blood collection bag 30 from flowing to the pre-processing part 16. Also, the blood-flow-path connector 10 of the present invention may be disposed on an end on the first bag 50 side of the first transfer tube 58 and on an end on the third bag 54 side of the third transfer tube 62 in the separation processing part 18 to keep blood components in the first bag 50 and the third bag 54 from being transferred to another bag.

In the above-mentioned embodiment, the blood-flow-path connector 10 is employed in the blood bag system 12 having a configuration in which the blood collection equipment 14 and the separation processing part 18 are originally connected as a unit, but the blood-flow-path connector 10 may also be employed in another type of blood-component collecting system where a part corresponding to the blood collection equipment 14 (blood collection system) and a part corresponding to the separation processing part 18 (separating system) are separated. In such blood-component collecting system, after whole blood is collected in the blood collection system, a blood collection bag (whole-blood bag) in the blood collection system and the separating system are connected, and then blood components such as platelets and red blood cells in the whole blood in the blood collection bag will be separated and collected. The blood-flow-path connector 10 may be disposed in a tube forming a blood-flow-path in such blood collection system or separating system.

Further, the blood-flow-path connector 10 of the present invention may be employed in a medical device including a flow-path for passing a fluid other than blood, and disposed in the flow-path.

In the above-mentioned embodiment, the blood-flow-path connector 10 connects the second end 70b of the communicating part 70 to a blood-flow-path upstream side side of blood flow, and the first end 68a of the tubular body 68 to a blood-flow-path downstream side; however, the first end 68a of the tubular body 68 may be connected to the blood-flow-path upstream side, and the second end 70b of the communicating part 70 may be connected to the blood-flow-path downstream side.

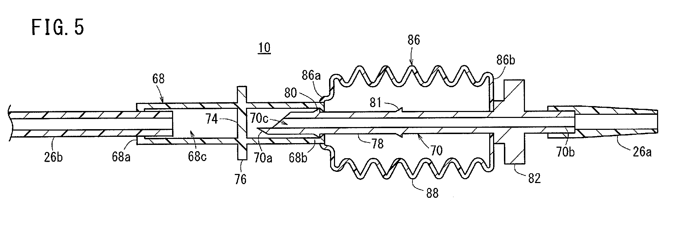

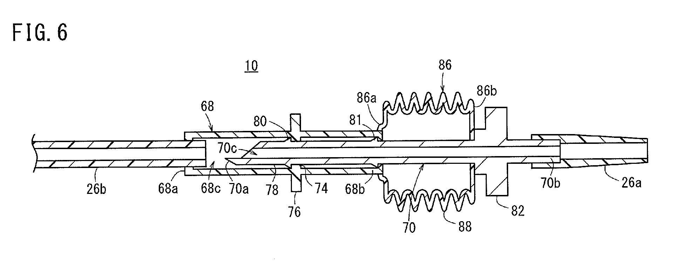

A modification of the blood-flow-path connector 10 according to the first embodiment will now be explained with reference to Figs. 5 and 6. The blood-flow-path connector 10 may be configured to include, instead of the above-mentioned the cover part 72 having a hollow cylindrical, ellipsoidal, or spherical shape, a cover part 86 having a main body 88 shaped into an axially contractible bellows, as shown in Figs. 5 and 6.

The bellows of the main body 88 of the cover part 86 is not contracted but keeps its original shape in an initial state before penetration of the blocking part 74, as shown in Fig. 5, but is axially contracted in a connected state after penetration of the blocking part 74, as shown in Fig. 6, reducing a distance between a first end 86a and a second end 86b thereof.

Thus, in the blood-flow-path connector 10 according to the modification, the cover part 86 having a bellows the main body 88 has substantially the same action and effect as the above-mentioned hollow cylindrical, ellipsoidal, or spherical the cover part 72; however, its bellows shape allows axial contraction with more ease compared to the above mentioned the cover part 72.

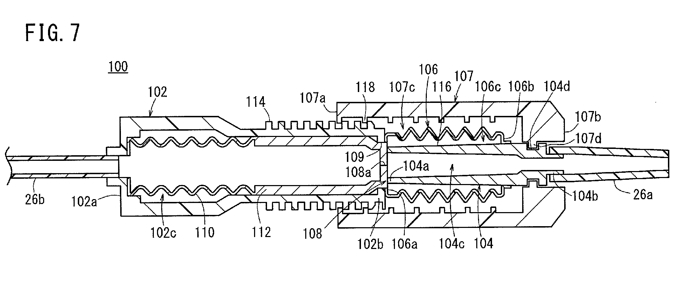

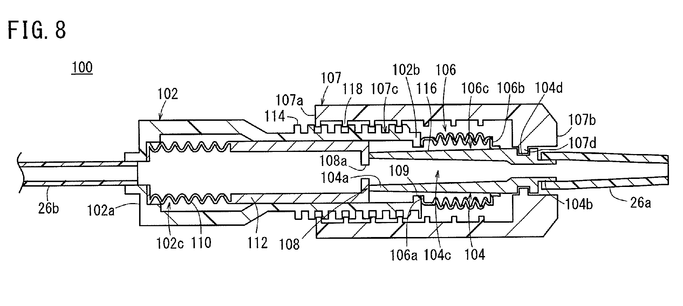

A blood-flow-path connector 100 according to a second embodiment of the present invention will now be explained with reference to Figs. 7 and 8. In the blood-flow-path connector 100 according to the second embodiment, an element having the identical or similar function and effect as that in the blood-flow-path connector 10 of the first embodiment will be assigned the same reference number, and will not be explained in detail.

As shown in Figs. 7 and 8, in the blood-flow-path connector 100, the blocking part 108 in a tubular body 102 is formed having a slit 108a, and as a communicating part 104 connected to a cover part 106 pushes the blocking part 108 in a pushing direction, the slit 108a is opened to open the blocking part 108. Further, the blood-flow-path connector 100 includes an operating part 107 that is axially movable relative to the tubular body 102 as screw-fitting to the tubular body 102.

The tubular body 102 is formed into an axially open, hollow cylindrical shape, and is configured such that the communicating part 104 is axially movable within its hollow section 102c. A first end 102a and a second end 102b of this tubular body 102 are both open relative to an axial direction. The first end 102a of the tubular body 102 is configured so that it can connect to a tube as a blood-flow-path; for example, it is connected to the second tube 26b that is on the blood collection bag 30 side of the second Blood collection tube 26. The second end 102b of the tubular body 102 is connected to a first end 106a of the cover part 106.

A non-limiting example of constituent material for the tubular body 102 is a resin material. The tubular body 102 may be configured substantially transparent so as to ensure the inner visibility.

Further, the tubular body 102 has the blocking part 108 that is axially movable within its hollow section 102c and a fitting part 109 for closely fitting the blocking part 108 at a predetermined position in the axial direction.

The blocking part 108 is for blocking a flow-path liquid-tightly in the hollow section 102c in the tubular body 102, and is shaped like, for example, a membrane or partition wall that divides the flow-path before and after itself. Around the center of the blocking part 108, the slit 108a is provided in a manner penetrating the blocking part 108 in an axial direction.

The fitting part 109 has an inner diameter allowing close fit of the blocking part 108; the hollow section 102c in the tubular body 102 on the first end 102a side relative to the fitting part 109 has an inner diameter larger than the inner diameter of the fitting part 109. Consequently, when the blocking part 108 is fitted to an inner circumference of the fitting part 109, in order for the fitting state to be close, the slit 108a is closed liquid-tightly; on the other hand, when the blocking part 108 is moved in a pushing direction and disjoined from the fitting part 109, the fitting state is loosened to deform the blocking part 108, and eventually the slit 108a is opened. The fitting part 109 is, for example, disposed at the second end 102b of the tubular body 102, as shown in Figs. 7 and 8.

In particular, the hollow section 102c in the tubular body 102 includes an axially contractible contracting part 110 on the first end 102a side, and a sliding part 112 on the second end 102b side, which is axially slidable within the hollow section 102c; an end of the sliding part 112 on the second end 102b side is formed as the blocking part 108. The contracting part 110 and the sliding part 112 are formed to be hollow, and these hollow sections are communicated with each other so as to constitute a blood-flow-path in the tubular body 102. The contracting part 110 and the sliding part 112 may be integrally formed.

The contracting part 110 is axially contractible, for example, it contracts in response to the sliding of the sliding part 112 in a pushing direction, securing a flow-path by means of a hollow section in the contracting part 110, whether before or after the sliding of the sliding part 112. The contracting part 110 may be one that is made of, for example, a bellows or flexible material.

The ends of the contracting part 110 are open, one of which is connected to the first end 102a of the tubular body 102 and the other of which communicates with the sliding part 112.

The sliding part 112 is axially slidable within the hollow section 102c in the tubular body 102, for example, it is pushed in a pushing direction within the hollow section 102c due to the movement of the communicating part 104 in the pushing direction. For the sliding part 112, one end to be in communication with the contracting part 110 is open, and the other end is formed as the blocking part 108 on the second end 102b side of the tubular body 102.

For a constituent material for the sliding part 112 including the blocking part 108, for example, a resilient material is preferably used, although there is no particular limitation as long as one is flexible and resilient to allow the slit 108a, that is normally closed liquid-tightly, to be opened as being pushed by the communicating part 104.

Further, the tubular body 102 is screw-fit to and axially movable relative to the operating part 107. For example, the tubular body 102 is configured to have an outer diameter smaller than an inner diameter of the operating part 107 so that it can be inserted into the operating part 107; a male thread part 114 is provided on an outer circumferential surface of the tubular body 102, which the male thread part 114 is screw-fit to a female thread part 118 provided on an inner circumferential surface of the operating part 107 to screw-fit and connect the tubular body 102 and the cover part 106.

The communicating part 104 has an axially open, hallow shape, which a hollow section 104c serves as a blood-flow-path, and a first end 104a and a second end 104b thereof are both open relative to an axial direction. This the communicating part 104 moves relative to the tubular body 102 in a pushing direction to push the sliding part 112 and the blocking part 108 within the hollow section 102c in the tubular body 102 in the pushing direction and disjoin the blocking part 108 from the fitting part 109, thereby opening a slit 108c to open the blocking part 108.

The communicating part 104 is configured to be axially movable within the hollow section 102c in the tubular body 102. For a constituent material for the communicating part 104, there is no particular limitation as long as one is capable of pressing the blocking part 108; for example, a resin material is used preferably. The communicating part 104 may be configured substantially transparent so as to ensure the inner visibility.

Further, the communicating part 104 includes a main body 116 on the first end 104a side, that is elongate enough to push the sliding part 112 and the blocking part 108. Here, the first end 104a of the communicating part 104, that is, a distal end of the main body 116, has a shape suitable for pressing the blocking part 108; for example, it may be configured to have a surface generating a suitable bearing surface pressure. The second end 104b of the communicating part 104 is configured so that it can connect to a tube as a blood-flow-path; for example, it is connected to the first tube 26a that is on the branch part 24 side in the second Blood collection tube 26.

The cover part 106 is a bag-like article having an axially open, hallow shape, and covers the communicating part 104 in a manner that the communicating part 104 is held in its hollow section 106c; and is provided on the second end 102b side of the tubular body 102. The cover part 106 may, for example, have a hollow cylindrical shape having the same axial direction as the blood-flow-path connector 100 or may have an ellipsoidal or spherical, or bellows shape.

The first end 106a and a second end 106b of the cover part 106 are both open relative to an axial direction. The first end 106a of the cover part 106 is connected to the second end 102b of the tubular body 102, and the other end, the second end 106b of the cover part 106 is connected to the communicating part 104, whereby the cover part 106 keeps the communicating part 104 clean, and even when a liquid leaks from the communicating part 104, the liquid is kept from leaking externally.

The cover part 106 is configured to be axially contractible so that it contracts when, for example, the tubular body 102 is inserted into the operating part 107, reducing a distance between the second end 102b of the tubular body 102 and a second end 107b of the operating part 107. The cover part 106 prevents a liquid from leaking from the communicating part 104 whether before or after the contraction. For a constituent material for the cover part 106, there is no particular limitation as long as one is axially contractible and flexible; for example, a resilient material or soft resin material is used preferably.

The operating part 107 has a hollow, axially open shape, and holds the communicating part 104 and the cover part 106 in hollow section 107c therein. Further, the operating part 107 is screw-fit to and axially movable relative to the tubular body 102. The operating part 107 is rotatably connected to the communicating part 104; thus, as the communicating part 104 moves axially, so does the operating part 107.

For example, a first end 107a of the operating part 107 is open, and the operating part 107 has an inner diameter larger than an outer diameter of the tubular body 102 such that the tubular body 102 can be inserted thereinto; further, an inner circumferential surface of the operating part 107 is provided with the female thread part 118, which the female thread part 118 is screw-fit to the male thread part 114 of the tubular body 102, to screw-fit the operating part 107 and the tubular body 102. The second end 107b of the operating part 107 is also open, and this opening portion is pivotally supported by the communicating part 104, whereby the operating part 107 is freely rotatable around an axis relative to the communicating part 104. In this embodiment, specifically, an outer circumference part of the base-end side of the communicating part 104 is provided with a circumferentially extended ring-shaped groove 104d, and an inner circumference part of the second end 107b of the operating part 107 is provided with a circumferentially extended ring-shaped projection 107d, wherein the ring-shaped projection 107d slidably and circumferentially fits to the ring-shaped groove 104d. Alternatively to this configuration, the outer circumference part of the base-end side of the communicating part 104 may be provided with circumferentially extended ring-shaped projection, and the inner circumference part of the second end 107b of the operating part 107 may be provided with circumferentially extended ring-shaped groove.

Accordingly, when the operating part 107 is rotated relative to the tubular body 102 to be moved in a pushing direction, the communicating part 104 is pushed by the operating part 107 to be moved in the pushing direction as well, and in turn the communicating part 104 pushes the sliding part 112 and the blocking part 108 in the pushing direction. Also, due to the screw-fit between the tubular body 102 and the operating part 107, the positions of the cover part 106 and the communicating part 104 relative to the tubular body 102 are sustained.

In the above-mentioned the blood-flow-path connector 100, as shown in Fig. 7, the blocking part 108 is closely fit to an inner circumference of the fitting part 109 before the communicating part 104 pushes the blocking part 108 in a pushing direction; therefore, the slit 108a is closed liquid-tightly. Accordingly, the flow-path before and after the blocking part 108 are divided by the blocking part 108, whereby the blood flow from the first Blood collection tube 22 to the second Blood collection tube 26 has been blocked.

Next, as shown in Fig. 8, the operating part 107 is rotated relative to the tubular body 102 to travel in a pushing direction so that the tubular body 102 is inserted deeply into the operating part 107, and then the communicating part 104 pivot ally-supported by the operating part 107 moves within the tubular body 102 in the pushing direction to push the sliding part 112 and the blocking part 108 in the pushing direction.

Here, because the blocking part 108 is moved in a pushing direction to be disjoined from the fitting part 109, a fitted state for the blocking part 108 is loosened and the blocking part 108 is deformed, and the slit 108a is opened to open the blocking part 108.

At this time in the tubular body 102, the contracting part 110 has been contracted and the cover part 106 has been contracted too.

Thus, with the blood-flow-path connector 100 according to the second embodiment as well as the blood-flow-path connector 10 according to the first embodiment, the fore-and-aft flow-path can be connected with easy operation, and an adequate blood-flow-path can be secured at the connection.

In the blood-flow-path connector 100 according to the second embodiment, by tightening the male thread part 114 of the tubular body 102 onto the female thread part 118 of the operating part 107, or by simply rotating the operating part 107 relative to the tubular body 102, the operating part 107 can be tightened against the tubular body 102 in a pushing direction, the communicating part 104 can push the blocking part 108, and the slit 108a can be opened to open the blocking part 108; in addition, positions of the operating part 107 and the communicating part 104 relative to the tubular body 102 can be sustained. Thus, according to the second embodiment,flow-path connection can be performed with easier operation.

Further, with the blood-flow-path connector 100 according to the second embodiment, the blocking part 108 can be opened without a need for breakage; therefore, a more adequate blood-flow-path can be secured. Furthermore, when tightening of the male thread part 114 against the female thread part 118 is loosened to move the operating part 107 relative to the tubular body 102 in an opposite direction to a pushing direction, the communicating part 104 is withdrawn from the tubular body 102, the contracting part 110 moves the sliding part 112 and the blocking part 108 to the opposite direction to the pushing direction by the repelling force of itself, the blocking part 108 is closely fit to an inner circumference of the fitting part 109, and the slit 108a is closed liquid-tightly. Thus, with the blood-flow-path connector 100 according to the second embodiment, reblocking of the flow-path is also possible.

It should be noted that, for those components in the second embodiment that are common with the first embodiment, the same or similar actions and effects as yielded by the common components in the first embodiment shall be yielded as a matter of course.

Needless to say, a blood-flow-path connector and blood bag system according to the present invention may employ various configurations other than the above-mentioned embodiments without departing from the spirit of the present invention.

## Claims

1. A blood-flow-path connector (10, 100), comprising:
a tubular body (68, 102) and a communicating part (70, 104) formed into a hollow shape, the hollow section (68c, 70c, 102c, 104c) having a flow-path for a fluid flowing axially therein, wherein
the tubular body (68, 102) has a first end (68a, 102a) constituting one end and a second end (68b, 102b) constituting the other end,
a direction from the second end (68b, 102b) side of the tubular body (68, 102) towards the first end (68a, 102a) side is defined as a pushing direction,
a blocking part (74, 108) is provided in the hollow section (68c, 102c) in the tubular body (68, 102) for blocking the flow of the fluid within the flow-path in the hollow section (68c, 102c),
the communicating part (70, 104) is disposed on the second end (68b, 102b) side of the tubular body (68, 102) and adapted to be axially movable within the hollow section (68c, 102c) in the tubular body (68, 102),
the blocking part (74, 108) is opened due to movement of the communicating part (70, 104) relative to the tubular body (68, 102) in the pushing direction, and
due to the opening of the blocking part (74, 108), the flow-path in the tubular body (68, 102) and the flow-path in the communicating part (70, 104) come to communicate with each other.

2. The blood-flow-path connector (10) according to claim 1, wherein
the hollow section (70c) in the communicating part (70) is the flow-path,
one end constituting the blocking part (74) side of the communicating part (70) is formed into a shape that is capable of penetrating the blocking part (74), and
the blocking part (74) is penetrated and opened by the communicating part (70) that has moved relative to the tubular body (68) in the pushing direction.

3. The blood-flow-path connector (10) according to claim 2, comprising:
a hollow cover part (72, 86) that is axially contractible and is capable of holding the communicating part (70) therein, wherein
afirstend (72a, 86a) of the cover part (72, 86) is connected to the tubular body (68) liquid-tightly, and
a second end (72b, 86b) of the cover part (72, 86) is connected to the communicating part (70) liquid-tightly and adapted to be axially movable together with the communicating part (70).

4. The blood-flow-path connector (10) according to claim 3, wherein
the cover part (86) is formed into a bellows.

5. The blood-flow-path connector (100) according to claim 1, wherein
the communicating part (104) is formed into a shape that is capable of pressing the blocking part (108),
the tubular body (102) comprises a fitting part (109) for closely fitting the blocking part (108) at a predetermined position in an axial direction, and
the blocking part (108) has a slit (108a) penetrating the blocking part (108) in an axial direction, and when the blocking part (108) is moved in the pushing direction and disjoined from the fitting part (109) by means of the communicating part (104) adapted to be axially movable within the tubular body (102) and has moved relative to the tubular body (102) in the pushing direction, the slit (108a) is opened to open the blocking part (108) .

6. The blood-flow-path connector (100) according to claim 5, wherein
the tubular body (102), within a hollow section (102c) thereof, comprises a hollow sliding part (112) that is axially slidable within the hollow section (102c) and a hollow contracting part (110) that is axially contractible, and
an end of the sliding part (112) on a second end (102b) side of the tubular body (102) is the blocking part (108), and each of the hollow sections of the sliding part (112) and the contracting part (110) is a flow-path in the tubular body (102).

7. The blood-flow-path connector (100) according to claim 5, comprising:
a hollow operating part (107) that is capable of holding the communicating part (104), wherein
the operating part (107) is screw-fit to and axially movable relative to the tubular body (102); and
the communicating part (104) is pivotally supported by the operating part (107) and moves along with the movement of the operating part (107) relative to the tubular body (102).

8. A blood bag system (12), comprising:
bags (30, 32, 50, 52, 54) for containing blood or blood components; and
tubes (22, 26, 28, 44, 46, 58, 60, 62) serving as flow-path for the blood or the blood components to the bags (30, 32, 50, 52, 54), wherein
a blood-flow-path connector (10, 100) is disposed for the tubes (22, 26, 28, 44, 46, 58, 60, 62),
the blood-flow-path connector (10, 100) includes a tubular body (68, 102) and a communicating part (70, 104) formed into a hollow shape, the hollow section (68c, 70c, 102c, 104c) having a flow-path for a fluid flowing axially therein,
the tubular body (68, 102) has a first end (68a, 102a) constituting one end and a second end (68b, 102b) constituting the other end,
a direction from the second end (68b, 102b) side of the tubular body (68, 102) towards the first end (68a, 102a) side is defined as a pushing direction,
the blocking part (74, 108) is provided in the hollow section (68c, 102c) in the tubular body (68) for blocking the flow of the fluid within the flow-path in the hollow section (68c, 102c),
the communicating part (70, 104) is disposed on the second end (68b, 102b) side of the tubular body (68, 102), adapted to be axially movable within the hollow section (68c, 102c) in the tubular body (68, 102),
the blocking part (74, 108) is opened due to movement of the communicating part (70, 104) relative to the tubular body (68, 102) in the pushing direction, and
due to the opening of the blocking part (74, 108), the flow-path in the tubular body (68, 102) and the flow-path in the communicating part (70, 104) come to communicate with each other.

9. The blood bag system (12) according to claim 8, wherein
the bags (30, 32, 50, 52, 54) include blood collection bag (30) for containing blood collected from a donor and a initially-drawn blood bag (32) for collecting initially-drawn blood during blood collection,
among the tubes (22, 26, 28, 44, 46, 58, 60, 62), a first Blood collection tube (22) to be connected to a blood collection needle (20) to be punctured into the donor is branched into a second Blood collection tube (26) to be connected to the blood collection bag (30) and a branch tube (28) to be connected to the initially-drawn blood bag (32), and
the blood-flow-path connector (10, 100) is disposed in the second Blood collection tube (26).
